# EUROPEAN PATENT SPECIFICATION

(11) **EP 1 278 517 B1**
(45) Date of publication and mention of the grant of the patent: **30.05.2007**
(21) Application number: 01918824.2
(22) Date of filing: 16.03.2001
(51) Int. Cl.: A61K 31/00, A61K 31/60, A61K 31/616, A61K 31/621, A61K 31/192, A61K 31/196, A61K 31/405, A61K 31/12, A61K 31/23, A61K 31/635, A61K 31/135, A61K 31/047, A61K 31/341, A61K 31/415, A61K 33/20

(54) **METHODS FOR IMPROVING SIZE AND APPEARANCE OF A WOUND**
VERFAHREN ZUR VERBESSERUNG DER GRÖSSE UND DES AUSSEHENS EINER WUNDE
PROCEDES PERMETTANT D'AMELIORER LA TAILLE ET L'ASPECT D'UNE BLESSURE

(30) Priority: 17.03.2000 US 190198 P
(43) Date of publication of application: 29.01.2003
(73) Proprietor: Avocet Polymer Technologies, Inc., Chicago, IL 60601 (US)
(72) Inventor: CAPELLI-SCHELLPFEFFER, Mary, Kenosha, WI 53144 (US)
(74) Representative: Kirkham, Nicholas Andrew
(86) International application number: PCT/US2001/008735
(87) International publication number: WO 2001/070210

(56) References cited:
- EP-A- 0 146 847
- WO-A-94/17839
- WO-A-98/07425
- DE-A- 2 707 537
- US-A- 4 346 108

## Description

### BACKGROUND OF THE INVENTION

Although scar formation and remodeling are essential processes in skin wound healing, disorders of excess scar formation, such as hypertrophic scars and keloids, remain a common clinical problem. A hypertrophic scar is an excessive wound scar which is thick and raised, having grown in size beyond that required for normal wound healing. A hypertrophic scar stays essentially within the boundaries of the original injury. A keloid is a raised scar that exceeds the boundaries of the initial injury, and which is rarely corrected by surgical intervention.

The changing patterns of the connective tissue matrix during repair following injury require a delicate balance between synthesis and degradation of collagen and proteoglycans. Under normal circumstances this balance is maintained, while in many diseased states it is altered, leading to an excessive deposition of collagen, to a loss of functional tissue, or to disfigurement. With hypertrophic scars and keloids, the biosynthetic phase continues longer than necessary to repair the wound. In order to maintain nutrient supply in hypertrophic scars and keloids scars, vascular in-growth occurs, resulting in a large, highly vascularized scars which are unsightly and can be disabling.

Existing therapy for hypertrophic scars and keloids includes surgery, mechanical pressure, steroids, x-ray irradiation and cryotherapy. There are many disadvantages associated with each of these methods. Surgical removal of the scar tissues is often incomplete and can result in further development of hypertrophic scars and keloids at the incision and suture points. Steroid treatments are unpredictable and often result in depigmentation of the skin. X-ray therapy is the only predictable effective treatment to date; however, because of its potential for causing cancer, it is not generally recommended or accepted. The most common approach to control hypertrophic scar and keloid formation is to apply pressure, which appears to be somewhat effective in many instances. However, this treatment has limited application, generally based on the size and location of the scar tissue on the body. Other commonly used treatments are application of Vitamin E and corticosteroids. Each of these agents can interfere with collagen synthesis and promote collagen degradation.

A need exists for improved methods of treating healed wounds such as scars.

### SUMMARY OF THE INVENTION

Essential and optional features of the present invention are set out in the accompanying main- and sub-claims respectively.

Embodiments of the present invention relates to compositions and uses thereof for the manufacture of a medicament for improving the size and appearance of a healed wound, which may be a scar such as, for example, a hypertrophic scar, a keloid, Dupuytren's contractures, acne scars, fibrotic scars, and reactive scars. The invention *inter alia* comprises the following, alone or in combination. Accordingly, the present invention relates to a use which includes administering to an individual having a healed wound or scar a therapeutically effective amount of a cyclooxygenase inhibitor, and may include the simultaneous administration of a substance such as an antiirritant, for example, diphenhydramine, to reduce skin irritation.

In one embodiment, the use includes contacting a healed wound with a thermal insulating material that elevates the surface temperature of the healed wound, and that includes an effective amount of at least one cyclooxygenase inhibitor. The thermal insulating material may also include a deodorant agent to reduce surface bacteria and odor formation. The thermal insulating material is allowed to remain in contact with the healed wound for a period of time sufficient to allow a noticeable improvement in its size and appearance.

Embodiments of the present invention also relate to a use for improving the size and appearance of a healed wound which includes administering to an individual having a healed wound an effective amount of at least one NF-kB inhibitor.

In yet another embodiment, a use for improving the size and appearance of a healed wound comprises contacting the healed wound with a thermal insulating material that elevates the surface temperature of the healed wound and that includes an effective amount of at least one NF-kB inhibitor, and allowing the thermal insulating material to remain in contact with the wound. The thermal insulating material including an NF-kB inhibitor is allowed to remain in contact with the healed wound for a period of time sufficient to allow a noticeable improvement in its size and appearance.

Embodiments of the present invention also relate to a use for improving the size and appearance of a healed wound comprising contacting the healed wound with a thermal insulating material that elevates the surface temperature of the healed wound, the thermal insulating material including at least one antiirritant compound; and allowing the thermal insulating material to remain in contact with the healed wound.

In another embodiment, a use for improving the size and appearance of healed wound includes contacting the healed wound with a hydrogel that elevates the surface temperature of the healed wound, the hydrogel containing an effective amount of acetylsalicylic acid. The hydrogel is allowed to remain in contact with the healed wound for a period of time sufficient to bring about an improvement in the healed wound, in particular an improvement in its size and appearance.

Another embodiment of the invention is a composition comprising up to about 35 percent of each of the following: salicylic acid or a derivative thereof; acetylsalicylic acid or a derivative thereof; a compound selected from aluminum hydroxide, aluminum zirconium trichlorohydrex, and other metallic anti-microbials; a compound selected from diphenhydramine and other anti-pruritic agents; a compound selected from ibuprofen and other non-steroidal agents specifically inhibiting prostaglandin E2; and a compound selected from non-steroidal agents specifically inhibiting cyclooxygenase 2.

In a particular embodiment, the composition comprises about 2 percent to about 5 percent of salicylic acid or a derivative thereof; about 2 percent to about 5 percent of acetylsalicylic acid or a derivative thereof; about 2 percent to about 5 percent of a compound selected from aluminum hydroxide, aluminum zirconium trichlorohydrex, and other metallic anti-microbials; about 2 percent to about 5 percent of a compound selected from diphenhydramine and other anti-pruritic agents; about 2 percent to about 5 percent of a compound selected from ibuprofen and other non-steroidal agents specifically inhibiting prostaglandin E2; and about 2 percent to about 5 percent of a compound selected from non-steroidal agents specifically inhibiting cyclooxygenase 2, and mixtures thereof. In one embodiment, the composition includes a therapeutically effective amount of a cyclooxygenase inhibitor such as salicylic acid or acetylsalicylic acid. The embodiment may further include a thermal insulating material. The present invention also encompasses a method for improving the size and appearance of a healed wound comprising administering these compositions to an individual in need thereof.

Another embodiment of the invention is a use improving the size and appearance of a healed wound comprising administering to an individual a composition comprising up to about 35 percent of each of the following: salicylic acid or a derivative thereof; acetylsalicylic acid or a derivative thereof; a compound selected from aluminum hydroxide, aluminum zirconium trichlorohydrex, and other metallic anti-microbials; a compound selected from diphenhydramine and other anti-pruritic agents; a compound selected from ibuprofen and other non-steroidal agents specifically inhibiting prostaglandin E2; and a compound selected from non-steroidal agents specifically inhibiting cyclooxygenase 2.

In a particular embodiment, the use comprises administering to an individual a composition comprising from about 2 percent to about 5 percent of salicylic acid or a derivative thereof; about 2 percent to about 5 percent of acetylsalicylic acid or a derivative thereof; about 2.percent to about 5 percent of a compound selected from aluminum hydroxide, aluminum zirconium trichlorohydrex, and other metallic anti-microbials; about 2 percent to about 5 percent of a compound selected from diphenhydramine and other anti-pruritic agents; about 2 percent to about 5 percent of a compound selected from ibuprofen and other non-steroidal agents specifically inhibiting prostaglandin E2; and about 2 percent to about 5 percent of a compound selected from non-steroidal agents specifically inhibiting cyclooxygenase 2, and mixtures thereof. Another embodiment includes administering the composition in a thermal insulating material.

Yet another embodiment of the invention includes the use, for the manufacture of a medicament for preventing or treating a condition caused by the appearance of a hypertrophic or a keloid scar on a healed wound, of an effective amount of an NF-kB inhibitor, or a cyclooxygenase inhibitor in combination with a substance that relieves skin irritation, an antimicrobal agent, and a thermal insulating material.

In another embodiment the invention comprises a kit for use in improving the size and appearance of a healed wound. A kit according to an embodiment of the invention may include a cyclooxygenase inhibitor or an NF-kB inhibitor and a hydrogel. In another embodiment, a kit may comprise a hydrogel that includes a cyclooxygenase inhibitor or an NF-kB inhibitor.

Another embodiment comprises a kit including a composition comprising up to about 35 percent of each of the following: salicylic acid or a derivative thereof; acetylsalicylic acid or a derivative thereof; a compound selected from aluminum hydroxide, aluminum zirconium trichlorohydrex, and other metallic anti-microbials; a compound selected from diphenhydramine and other anti-pruritic agents; a compound selected from ibuprofen and other non-steroidal agents specifically inhibiting prostaglandin E2; and a compound selected from non-steroidal agents specifically inhibiting cyclooxygenase 2.

In a particular embodiment, a kit includes a hydrogel and a composition comprising about 2 percent to about 5 percent of salicylic acid or a derivative thereof; about 2 percent to about 5 percent of acetylsalicylic acid or a derivative thereof; about 2 percent to about 5 percent of a compound selected from aluminum hydroxide, aluminum zirconium trichlorohydrex, and other metallic anti-microbials; about 2 percent to about 5 percent of a compound selected from diphenhydramine and other anti-pruritic agents; about 2 percent to about 5 percent of a compound selected from ibuprofen and other non-steroidal agents specifically inhibiting prostaglandin E2; and about 2 percent to about 5 percent of a compound selected from non-steroidal agents specifically inhibiting cyclooxygenase 2, and mixtures thereof.

### DETAILED DESCRIPTION OF THE INVENTION

A description of preferred embodiments of the invention follows.

Embodiments of the present invention relate to compositions and uses thereof for the manufacture of a medicament for improving the size and appearance of a healed wound or a scar, and for reducing scar irritation. In one embodiment, the present invention provides methods and compositions for the specific inhibition of one distinct pathway in cyclooxygenase regulation, thereby inhibiting the expression of skin irritation and excessive scar symptoms in a healed wound, while leaving balance in other aspects of cyclooxygenase modulation.

EP-A-0 146 847 discloses the use of salicylic acid for preventing the formation of keloids applied to burns to promote re-epitheliazation.

US-A-4 346 108 teaches that ibuprofen can be used for prevention of scar tissue. US-A-4 346 108 teaches system administration of ibuprofen prior to surgery.

DE 2707537 relates to the use of salicylic acid for the local treatment or control of hypertrophic cicatrization in acne.

WO-A-98/07425 teaches that TNF plays a role in mediating or exacerbating keloid or scar formation and that application of a 4, 5 substituted imidazole can inhibit TNF production.

WO-A-94/17839 discloses the us of thermal insulating materials for the treatment of fibromatosis, keloids, hypertrophic wounds etc.

The compositions and uses as mentioned above according to embodiments of the invention can be used on any vertebrate with skin. Examples of such vertebrates include mammals (for example, human, bovine, porcine, canine, feline) and avian.

As used herein, the terms "healed wound" or "scar" mean a closed wound, such as a wound surface that is closed by regrowth of an epithelial barrier. A wound is "closed" after an open wound has been re-epithelialized. Closed wounds can result in the formation of a scar, which is never an exact replacement of the original tissue. Scar tissue is less elastic than the undamaged tissue and has surface and contour irregularities. As used herein, the term "affected area of skin" may also be used to refer to either of the teens "healed wound" or "scar."

In one embodiment, a healed wound is an area of skin that has pain, tingling, burning, and/or itching. In another embodiment, a healed wound is a scar. In another embodiment, a scar is an area of skin that has pain, tingling, burning, itching, discoloration, surface irregularities, and/or an erratic accumulation of fibrous tissue.

A wound may result from any of a number of types of skin traumas such as laceration, avulsion, burn, surgery, infection, acne, chemical facial peel, and accident An open wound closes by regrowth of an epithelial barrier, the regrowth replacing some of the normal tissue which had been destroyed by trauma. Sometimes, in the healed wound or scar, excessive and disfiguring deposits of fibrous tissue having an erratic accumulation of collagen occur.

One such scar which can result from an overproduction of collagen and excess deposition of scar tissue is a hypertrophic scar. As used herein, the term "hypertrophic scar" includes a scar characterized by thick, raised scar tissue that stays essentially within the boundaries of the original injury. Hypertrophic scars contain characteristic nodules, and result from a full-thickness injury, such as a surgical incision on skin. These scars' can cause problems such as aesthetic deformity and severe limitation of motion. For example, an excessive post-operative scar can develop as a result of "over-healing" or hypertrophic healing of a post-operative site.

Another type of scar in which there is an excess deposition of scar tissue is called a "reactive scar." As the term is used herein, a reactive scar is a normal, healed scar which, through mechanical disruption such as scratching or other irritation, is actively producing a hypertrophic tissue response.

Excessive scar deposition also occurs in a "fibrotic scar." As the term is used herein, a fibrotic scar is an accumulation of irritated fibrotic tissue at the site of a healed injury which may or may not have involved an observable wound.

Another type of scar that can result from an excess deposition of scar tissue is a keloid. As used herein, the term "keloid" includes a scar characterized by thick, raised scar tissue that exceeds the initial boundaries of the trauma and that lacks nodules. In contrast to hypertrophic scars, keloids proliferate beyond the wound edges, can result from superficial injuries, and are rarely treated successfully by surgery. Keloids frequently develop after bums, particularly where the skin is under tension, such as on the breastbone.

Another type of scar tissue that may be treated by embodiments of the method and compositions of the present invention is Dupuytren's contracture. Dupuytren's contracture arises from unknown causes and is a progressive, scar-like shrinkage and thickening of the flexion contracture of the cusp-like extended palmar aponeurosis in the palm of the hand, whereby, as the curvature of the fingers increases, especially that of the fourth and fifth fingers, stretching of the fingers becomes ever more restricted. This ailment, which attacks men more frequently than women and can occur in one or both hands, begins with a dimple-like indentation in the palm of the hand and gradually but quite painlessly grows into nodules and fascicles. The flexor tendons of the fingers concerned are not in themselves diseased but their movement is impaired by the scar-fascicles of the palmar aponeurosis. A similar contracture concerning the toes is known.

Since the illness neither regresses spontaneously nor responds with any degree of long term success to conventional forms of treatment (without surgery) such as massage, heat treatment and the like, it can only be treated surgically, namely, by cutting away the proliferating atrophied tissue. In addition to ordinary risks and unpleasantness associated with any surgical operation, there exists a further risk that scars resulting from the operation can make a later recurrence of the ailment even worse.

One embodiment of the present invention is based, in part, on the discovery that the size and appearance of a healed wound (e.g., a scar) can be improved, and the discomfort, itching, pain, and /or other symptoms caused by excessive tissue growth in a healed wound can be alleviated (partially or completely) by the administration of at least one compound that inhibits (partially or completely) cyclooxygenase, (e.g., cyclooxygenase-1, cyclooxygenase-2) directly or indirectly. For example, the function and/or expression of cyclooxygenase can be inhibited.

By "direct or indirect inhibition of cyclooxygenase" is meant that some compounds used according to an embodiment act directly on cyclooxygenase, while some compounds act indirectly on cyclooxygenase by acting on an upstream or downstream target in the cyclooxygenase pathway. For example, cyclooxygenase-2 can be inhibited indirectly by inhibiting, e.g., NF-kappa B (NF-k B) or adenosine. Further, there are some compounds that inhibit the function of cyclooxygenase both directly and indirectly, and are therefore more powerful or effective in treating the size and appearance of a healed wound than those compounds that have only one mode of action.

Cyclooxygenase-2 promotes pain, irritation, overgrowth of scar tissue and other scar symptoms that can lead to hypertrophic scars or keloids. Both cyclooxygenase-1 and cyclooxygenase-2 are enzymes that help produce hormones called prostaglandins. There is evidence that prostaglandins made by cyclooxygenase-2 lead to pain, irritation and excessive or abnormal scar growth.

Thus, in one embodiment of the invention, the compositions and methods described herein to inhibit cyclooxygenase directly are believed to specifically act on cyclooxygenase itself. Examples of compounds that directly inhibit cyclooxygenase include compounds such as salicylic acid; acetylsalicylic acid; aryl, substituted or unsubstituted aralkyl, allyl, and substituted or unsubstituted, linear, branched, or cyclic alkyl esters of salicylic acid; aryl, substituted or unsubstituted aralkyl, allyl, and substituted or unsubstituted, linear, branched, or cyclic alkyl esters of acetylsalicylic acid; ibuprofen; celecoxib; rofecoxib; flufenamic acid; indomethacin; nabumetone; naproxen; pharmaceutically acceptable salts thereof; and blends thereof. Celecoxib is chemically designated as 4-(5-(4-methylphenyl)-3-(trifluoromethyl)-1H-pyrazol-1-yl) benzenesulfonamide, and is a diaryl substituted pyrazole, with a molecular weight of 381.38. Rofecoxib is chemically designated as 4-(4-(methylsulfonyl)phenyl)-3-phenyl-2(5H)-furanone, and has a molecular weight of 314.36.

Flufenamic acid is chemically designated as (N*-*(α,α,α*-*trifluoro*-m-*tolyl) anthranilic acid), and has a molecular weight of 281.23.

Indomethacin is chemically designated as (1-(p-chlorobenzoyl)-5-methoxy-2-methylindole-3-acetic acid), and has a molecular weight of 357.8.

Nabumetone is a naphthylalkanone chemically designated as 4-(6-methoxy-2-naphthalenyl)-2-butanone, and has a molecular weight of 228.3.

Naproxen is chemically designated as (S)-(+)-6-methoxy-α-methyl-2-naphthaleneacetic acid, and has a molecular weight of 230.27.

In a preferred embodiment, an ester of acetysalicylic acid such as, methyl acetylsalicylate, ethyl acetylsalicylate, allyl acetylsalicylate, and benzyl acetylsalicylate, is used to inhibit cyclooxygenase.

In another preferred embodiment, sodium salicylate is used.

In another embodiment, the compositions and methods described herein inhibit cyclooxygenase indirectly and can work on a compound upstream or downstream of the cyclooxygenase pathway. As this molecular pathway is elucidated, the present invention provides an appropriate intervention in the cascade of reactions which leads to excessive scar growth and related symptoms. In a particular embodiment, the composition which indirectly inhibits upstream of the cyclooxygenase pathway is an inhibitor of NF-kB.

NF-kB is a signaling molecule that is detected in the body through biotechnology techniques. The results of the work described herein indicate that NF-kB plays a critical role in a biochemical mechanism that promotes irritation in healed wounds and leads to the symptom complex of excessive scar growth, pain and pruritis. NF-kB is a transcription factor, a eukaryotic protein that promotes RNA polymerase to recognize promoters. The structure of NF-kB is that of a heteromeric complex, that is, a molecule made up of sub-units. When the sub-units remain intact, NF-kB resides in the cytoplasm, where it exists in an inactive form, bound to I-kB, an inhibitory protein. A stimulant can trigger release of NF-kB from I-kB, whereupon the NF-kB moves from the cytoplasm to the nucleus, binds to DNA, and regulates transcription of specific genes. NF-kB binding sites are present on genes which direct the expression of proteins including cytokines and adhesion molecules. NF-kB is believed to activate a number of genes involved in immune and irritation responses.

When released, NF-kB is believed to signal the induction of cyclooxygenase, which leads to irritation of a healed wound or scar, and excessive growth of the scar. Thus, NF-kB appears to provide the primary mechanism that promotes increased rRNA2, prostaglandinE2, and the symptom complex including excessive scar growth, irritation, pain and pruritis.

Although the exact mechanisms of pharmacological inhibition of cyclooxygenase and of NF-kB are not fully understood, it appears that one molecular pathway, for example, is the inhibition, by acetylsalicylic acid, of NF-kB's translocation to the nucleus. Another possible mechanism includes inhibition by acetylsalicylic acid of IkB phosphorylation and catabolism. Because phosphorylation and degradation of IkB permit translocation of NF-kB to the nucleus, inhibition of these processes interferes with the movement of NF-kB from the cytoplasm to the nucleus, and ultimately, the activation of genes involved in immune responses and in responses to irritants. In yet another possible mechanism, salicylic acid and other salicylates may cause cells to release small amounts of adenosine, an anti-irritant.

Thus, in a particular embodiment, the invention includes an indirect inhibitor of cyclooxygenase that is an inhibitor of NF-kB. Examples of inhibitors of NF-kB that can be used according to this embodiment of the invention include the following compounds: salicylic acid; acetylsalicylic acid; aryl, substituted or unsubstituted aralkyl, allyl, and substituted or unsubstituted, linear, branched, or cyclic alkyl esters of salicylic acid; aryl, substituted or unsubstituted aralkyl, allyl, and substituted or unsubstituted, linear, branched, or cyclic alkyl esters of acetylsalicylic acid; nabumetone; sulindac sulfide; sulindac sulfone; sulfasalazine; and pharmaceutically acceptable salts thereof; and blends thereof. In another embodiment, a pro-drug may be used.

Sulfasalazine is chemically designated as (5-(4-(2-pyridylsulfamoyl)phenylazo)salicylic acid), and has a molecular weight of 398.40.

In a preferred embodiment, the NF-kB inhibitor is an ester of acetylsalicylic acid such as methyl acetylsalicylate, ethyl acetylsalicylate, allyl acetylsalicylate, or benzyl acetylsalicylate.

In another preferred embodiment the NF-kB inhibitor is sodium salicylate.

By "improving" the size and appearance of a healed wound or a scar according to an embodiment of the invention is meant alleviating, either partially or completely, symptoms such as pain, tingling, itching, burning, discoloration; reducing the size of a scar; reducing surface irregularities; reducing the accumulation of fibrous tissue; and/or partially or completely eliminating the scar.

In one embodiment of the invention, the composition is used to relieve or to prevent a condition of scar irritation, in particular in a case wherein scar irritation leads to symptoms including itching, and to a patient's self-inflicted mechanical action of scratching, which can result in further scar irritation, and possible contamination and invasion of the scar with native skin organisms.

### Administration

To achieve the improvements described herein, the present invention provides for a route of administration of the cyclooxygenase inhibitor or the NF-kB inhibitor that is either a topical or transdermal administration, an oral administration, an administration by injection, an administration by inhalation, or that is a combination of two or more of these administration routes.

While a composition for use in improving the size and appearance of a healed wound or scar may be administered in the form of a raw chemical compound, including a physiologically acceptable salt of the active ingredient, it is preferred to introduce the active ingredient in a pharmaceutical composition together with one or more adjuvants, excipients, carriers and/or diluents.

Therefore, in another embodiment, the present invention also relates to pharmaceutical compositions which include a suitable pharmaceutical carrier and at least one of the following: a cyclooxygenase inhibitor, an NF-kB inhibitor, an anti-irritant substance, a deodorant agent, aluminum hydroxide, and an anti-microbial substance such as aluminum zirconium trichlorohydrex, or other metallic anti-microbial. Any of the compositions of the present invention described herein may be administered with a suitable pharmaceutical carrier, the choice of which depends on the route of administration and the size of the scar. The terms "suitable pharmaceutical carrier," "pharmaceutically acceptable," and grammatical variations thereof, as they refer to compositions, carriers, diluents and reagents, are used herein interchangeably. As the terms are used herein, "suitable pharmaceutical carrier" and "pharmaceutically acceptable" refer to non-toxic materials that do not interfere with the effectiveness of the biological activity of active ingredients, and represent that the materials are capable of administration to or upon a vertebrate with a minimum of undesirable physiological effects such as nausea, dizziness, gastric upset and the like. The characteristics of the carrier will depend on the route of administration.

The preparation of a pharmacological composition that contains active ingredients dissolved or dispersed therein is well understood in the art and need not be limited based on formulation. Liquid preparations include solutions, suspensions, colloids, hydrogels, and emulsions, for example, water or water-propylene glycol mixtures. Such compositions may be prepared as injectables, either as liquid solutions or suspensions. Solid forms suitable for dissolving in a hydrogel or a liquid solution, or for suspending in liquid prior to use, can also be prepared. The preparation can also be emulsified. The active ingredient can be mixed with excipients which are pharmaceutically acceptable and compatible with the active ingredient and in amounts suitable for use in the therapeutic methods described herein. Suitable excipients include, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient. Details on techniques for formulation and administration may be found in the latest edition of *Remington's Pharmaceutical Sciences* (Maack Publishing Co., Easton, Pa.).

At least one cyclooxygenase inhibitor, or at least one NF-kB inhibitor, or combinations thereof, may be administered topically, orally, by injection, by inhalation, or by any suitable combination of these methods of administration.

### Topical Administration

For topical or transdermal administration, one embodiment of the invention encompasses placing the cyclooxygenase inhibitor or the NF-kB inhibitor directly on the surface of the scar or healed wound. A composition according to an embodiment of the invention is not intended for use on infected skin or on open wounds. In a particular embodiment, the cyclooxygenase inhibitor or the NF-kB inhibitor are mixed with a suitable pharmaceutical carrier and then placed directly on the surface of the affected area of skin. In another embodiment, a composition for use in improving the size and appearance of a healed wound or scar is placed in contact with the affected area of skin; the composition is then covered with a thermal insulating material. In each embodiment described herein, the composition placed in contact with the affected area of skin is allowed to remain in place for a period of time sufficient to bring about an improvement in the size and appearance of the healed wound or scar.

Although there is no minimum time required for duration of use, the duration of use of a composition of the present invention can extend for example, from about 0.5 hour to about 24 hours, or from about 1 hour to about 1 month, or from about 8 hours to about 12 months or from about 24 hours to about 24 months. In one embodiment of the invention, the duration of use typically extends from about 12 hours to about 12 months, for separate time periods. The composition can be used continuously or intermittently for a particular time period and then removed or reapplied. For example, the composition can be used intermittently from about 1 hour to about 72 hours, from about 8 hours to about 48 hours, from about 12 hours to about 24 hours, or from about 18 hours to about 24 hours. The time interval between each use can be from about 1 hour to about 72 hours, from about 8 hours to about 48 hours, from about 12 hours to about 24 hours, or from about 18 hours to about 24 hours. In a particular embodiment, each time period is about 18 hours in a day with a minimum of about 4 hours between time periods.

In one embodiment in which topical administration is used to administer a composition according to an embodiment of the invention, the use of adhesive tape is avoided because adhesives may cause irritation of a scar and aggravate the scar condition. It is recommended that means such as flexible wraps, elastic garments, netting, ace wraps or spandex sleeves or garments be used to affix a composition according to the invention to the affected area of skin.

In another embodiment, the invention encompasses including a cyclooxygenase inhibitor or an NF-kB inhibitor in a thermal insulating material. As used herein, the term "thermal insulating material" includes materials that, when placed in contact with or near to the skin, are capable of retaining sufficient heat to elevate the surface temperature of the affected area of the skin.

In one embodiment of the invention, the thermal insulating material when in use to cover the affected area, causes an elevation in the surface temperature of the healed wound or scar of from about 0.5°C to about 5°C. In another embodiment, the thermal insulating material, when used to cover the affected area, causes an elevation in the surface temperature of the healed wound or scar of from about 1°C to about 4°C. In a preferred embodiment, the thermal insulating material, when used to cover the affected area, causes an elevation in the surface temperature of the healed wound or scar of from about 2°C to about 3°C.

In one embodiment, the thermal insulating material may be a sponge. Examples of sponge materials suitable for use as a thermal insulating material in the present invention include collagen and cross-linked collagen. The term "cross-linked," as used herein, refers to covalent bonds formed among polymeric chains and to an interconnected structure wherein cross-links are formed between hydrophobic molecules, between hydrophilic molecules and between hydrophobic molecules and hydrophilic molecules.

In another embodiment, the thermal insulating material may be a gel, a hydrogel, or a biodegradable hydrogel. Gels and hydrogels generally contain a very high concentration of water, e.g., about 60% to about 98% water and are held together by a variety of cellular groups. The water may be bound in the form of various hydrates, or unbound, entrapped in cellular pockets formed by the polymer network groups.

The term "hydrogel" is used herein to mean a polymeric material which can include a cross-linked macromolecular network, which exhibits the ability to swell in water and to retain a significant portion of water within its structure without dissolving.

A "biodegradable hydrogel," as the term is used herein, is a hydrogel formed from a hydrogel-forming system containing at least one biodegradable component, i.e., a component which is degraded by water and/or by enzymes found in nature.

There are a number of well-known hydrophilic, polymeric compounds both naturally occurring and synthetic, which form networks, creating a gel in the presence of water. For example, gelatin can be obtained from the hydrolysis of collagen by boiling skin, ligaments, tendons, etc. A mixture of only 2% gelatin in water will form a stiff gel. An example of a gel suitable for use in an embodiment of the invention is Elastogel®, available from Southwest Technologies, Kansas City, MO.

A hydrogel may be formed by adding a solute such as gelatin to water at an elevated temperature to dissolve gelatin. The solution is then cooled and the solute(s) (e.g., solid gelatin components) form submicroscopic crystalline particle groups which retain a great deal of water in the interstices (so-called "brush-heap" structure). Methods of making hydrogels suitable for use in the present invention are well-known to those of skill in the art. See, for example, the disclosures of U.S. Patent Numbers 4,646,730 to Schonfeld *et al.;* 5,013,769 to Murray *et al.;* 4,659,700 to Jackson *et al.;* and 4,909,244 to Quarfoot *et al..* An example of a hydrogel suitable for use in an embodiment of the invention is AVOGEL®, available from Avocet Polymer Technologies, Inc., Chicago, IL.

In addition to increasing the surface temperature of the healed wound, the thermal insulating material may also be used to deliver a therapeutically effective substance to the healed wound. As used herein, the terms "therapeutically effective substance" or "therapeutic substance" include:
(i) Compounds and compositions recognized in the official United States Pharmacopoeia, the official Homeopathic Pharmacopoeia of the United States, or the official National Formulary, or any supplement of any of them;
(ii) Compounds and compositions intended for use in the diagnosis, cure, mitigation, treatment, or prevention of disease in man or other animals; and
(iii) Compounds and compositions (other than food) intended to affect the structure or any function of the body of man or other animals.

Examples of therapeutically effective substances include cyclooxygenase inhibitors; NF-kB inhibitors; substances that relieve skin irritation, such as glyceryl monooleate, diphenhydramine, calamine, and C₃ C₄ diols; deodorant agents such as aluminum zirconium trichlorohydrex and zinc acetate; aluminum hydroxide and anti- microbials; and ibuprofen and other non-steroidal agents specifically inhibiting prostaglandinE2.

In one embodiment, one or more therapeutically effective substances may be applied to one surface of the thermal insulating material. The thermal insulating material is then applied to the healed wound in a manner such that the therapeutically effective substance is placed in contact with the healed wound.

In another embodiment, the therapeutically effective substance is dispersed within a hydrogel, a water-insoluble gel, or sponge. The hydrogel, water-insoluble gel, or sponge within which the therapeutically effective substance is dispersed, is then placed in contact with the affected surface of the skin, and allowed to remain in place for a period of time sufficient to bring about an improvement in the size and appearance of the healed wound.

As used herein, the term "dispersed" includes ionic, covalent, hydrophilic, or hydrophobic interactions between the therapeutically effective substance and the hydrogel, water-insoluble gel, or sponge.

For example, a therapeutically effective substance containing a cationic moiety can be immobilized on a hydrogel polymer chain. As will be recognized by those skilled in the art, this cationic site may serve as a noncovalent, ionic binding site for anionic substances, such as certain non-steroidal drugs.

In another example, a hydrogel or sponge can be chosen which covalently bonds to the therapeutic substance used according to one embodiment.

Through hydrophilic interactions with water in the hydrogel, any water soluble drug will dissolve in the hydrogel.

A hydrophobic interaction between a non-water soluble therapeutic substance and a hydrogel can occur when the hydrogel selected includes a hydrophobic entity which is receptive to further interaction with a therapeutic substance having a hydrophobic moiety.

One skilled in the art will know, or will be able to ascertain with no more than routine experimentation, what hydrogels are suitable for dispersing a particular therapeutic substance.

A therapeutic substance which covalently bonds to the hydrogel or sponge can form a drug delivery substance with controlled or sustained release. If a biodegradable hydrogel or sponge is used, delivery of the therapeutic substance to the healed wound or scar is also related to the rate of degradation of the hydrogel or sponge. The degradation rate of the hydrogel or sponge is usually slower than the diffusion rate of the therapeutic substance. As is well-known to those of skill in the art, by choosing a particular concentration of each therapeutic substance used in a particular embodiment, and a particular hydrogel or sponge, one can control the rate of degradation or the rate of diffusion, and thus, the rate of delivery of the therapeutic substance.

The hydrogel or other thermal insulating material containing the therapeutically effective substance can remain in contact with the surface of the affected area of skin for about between 0.5 to about one hour per day, from about one hour to about 8 hours per day, from about 12 hours to about 15 hours per day, from about 12 hours to about 18 hours per day, from about 18 hours to about 24 hours per day, or over a number of days, for a sufficient number of days to bring about an improvement in the size and appearance of the healed wound or scar. The thermal insulating material can be removed periodically in order to cleanse the scar surface and to apply a fresh sample of therapeutically effective substance and thermal insulating material.

In one embodiment, at least one cyclooxygenase inhibitor, or at least one NF-kB inhibitor, or combinations thereof, are administered topically with a suitable pharmaceutical carrier, including one or more substances that relieve skin irritation. In a particular embodiment of the invention wherein the method of administration is topical, the substance that relieves skin irritation includes at least one of the following substances: glyceryl monooleate, diphenhydramine, calamine, and a C₃-C₄ diol.

In one embodiment, a healed wound, such as a scar, is contacted with a hydrogel comprising at least one a cyclooxygenase inhibitor, for example, ibuprofen, indomethacin, sodium salicylate, or at least one NF-kB inhibitor, for example, acetyl salicylic acid, sulfasalazine, or combinations thereof, and a deodorant agent to reduce surface bacteria and odor formation.

In one embodiment, a healed wound is treated by contacting the healed wound with a hydrogel that elevates the surface temperature of the affected area of skin, the hydrogel including an effective amount of acetylsalicylic acid in a suitable pharmaceutical carrier. The hydrogel is allowed to remain in contact with the affected area of skin for a period of time sufficient to result in an improvement in the healed wound.

Examples of suitable patterns of use according to an embodiment of the invention include, among others: use of various hydrogel combinations in sequence; use of various hydrogel combinations simultaneously; use of various hydrogel combinations in systemic-topical co-administration, such as oral administration simultaneouly with topical administration; use of combinations of active ingredients mixed by a pharmacist according to a prescription; and use of combinations of separate active ingredients available in kit form, mixed by the patient and self-administered according to physician instructions or directions provided with the kit.

Examples of various hydrogel combinations, that is, hydrogel combined with one or more active ingredients, that may be used according to an embodiment of the invention are provided in the Table below. Many other combinations are possible, and the examples are not to be construed as limiting the scope of the invention.

**Key for Table:**

| | |
|---|---|
| AL - aluminum zirconium trichlorohydrex | NM - Nabumetone |
| ASA - acetylsalicylic acid | NaSal -sodium salicylate |
| C - calamine | NX - naproxen |
| D - diphenhydramine | Sul - sulindac sulfide |
| FA - flufenamic acid | Ssne - sulindac sulfone |
| Ibu - ibuprofen | Ssz - sulfasalazine |
| IN - indomethacin | Zn - zinc acetate |
| | ✔ - indicates use of ingredient |

**Table**

| Hydrogel for Scar Control, Examples of Multiple Combinations | | | | | | | | | | | | | | | | | |
|---|---|---|---|---|---|---|---|---|---|---|---|---|---|---|---|---|---|
| Hydrogel | Antipruritic (a) | | Inhibitors of Cyclooxygenase | | | | | | | Inhibitors of NF-kB | | | | | | Antibacterials | |
| | C | D | ASA | FA | IN | NM | N X | Ibu | NaSal | NM | ASA | NaSal | Ssne | Sul | Ss z | AL | Zn |
| 1 | | | ✔ | | | | | | | | ✔ | | | | | | ✔ |
| 2 | | ✔ | ✔ | | | | | | | | ✔ | | | | | | |
| 3 | | ✔ | ✔ | | | | | | | | ✔ | | | | | | ✔ |
| 4 | | ✔ | ✔ | | | | | | | | ✔ | | | | | ✔ | |
| 5 | ✔ | | ✔ | | | | | | | | ✔ | | | | | | |
| 6 | | ✔ | | | | ✔ | | | | ✔ | | | | | | ✔ | |
| 7 | | ✔ | | | | ✔ | | | | ✔ | | | | | | | ✔ |
| 8 | | | | | | | ✔ | | | | | | | | | ✔ | |
| 9 | | | | | | | ✔ | | | | ✔ | | | | | ✔ | |
| 10 | | ✔ | | | | | ✔ | | | | | ✔ | | | | ✔ | |

### Oral and Parenteral Administration

In addition to compositions suitable for topical or transdermal administration to the affected area of skin, in another embodiment of the invention, compositions may be those suitable for oral or parenteral (including intramuscular, sub-cutaneous and intravenous) administration, or those in a form suitable for administration by inhalation.
The therapeutically effective substance of the invention, together with a conventional adjuvant, carrier, or diluent, may thus be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use; or in the form of sterile injectable solutions for parenteral (including sub-cutaneous) use.

In one embodiment of the invention, aqueous solutions suitable for oral use can be prepared by dissolving the active component in water and adding suitable colorants, flavors, stabilizing and thickening agents, as desired. Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like. Solid form preparations include, among others, powders, tablets, pills, capsules, and dispersible granules. A solid carrier can be one or more substances which may also act as diluents, flavoring agents, solubilizers, lubricants, suspending agents, binders, preservatives, tablet disintegrating agents, or an encapsulating material. In powders, the carrier is a finely divided solid which is in a mixture with the finely divided active component. In tablets, the active component is mixed with the carrier having the necessary binding capacity in suitable proportions and compacted in the shape and size desired.

According to an embodiment of the invention, powders and tablets preferably contain from five or ten to about seventy percent of the active compound. Suitable carriers are magnesium carbonate, magnesium stearate, sugar, lactose, pectin, dextrin, starch, gelatin, tragacanth, methylcellulose, sodium carboxymethylcellulose, a low melting wax, cocoa buffer, and the like. The term "preparation" is intended to include the formulation of the active compound with encapsulating material as carrier providing a capsule in which the active component, with or without carriers, is surrounded by a carrier, which is thus in association with it. Similarly lozenges are included. Tablets, powders, capsules, pills and lozenges can be used as solid forms suitable for oral administration.

According to an embodiment of the invention, liquid preparations include solutions, suspensions, and emulsions, for example, sterile water or water-propylene glycol solutions. For example, parenteral injection liquid preparations can be formulated with polyethylene glycol in aqueous solution. Other suitable pharmaceutical carriers for parenteral administration include, for example, physiological saline, bacteriostatic saline (saline containing about 0.9% mg/ml benzyl alcohol), phosphate-buffered saline, Hank's solution, Ringer's-lactate and the like. An embodiment of a therapeutically effective substance according to the present invention may thus be formulated for parenteral administration (by injection, for example, by bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilizing and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

### Administration by Inhalation

According to an embodiment of the invention, administration may also be made to the respiratory tract by means of an aerosol formulation in which the active ingredient is provided in a pressurized pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The doseage of the therapeutic substance may be controlled by provision of a metered valve. In compositions intended for administration to the respiratory tract, including intranasal compositions, compounds used in an embodiment will generally have a small particle size, for example of the order of 5 microns or less. Such a particle size may be obtained by means known in the art, for example by micronization.

### Therapeutically Effective Amount and Dosage

As used herein, the terms "therapeutically effective amount" and "therapeutically effective dose" refer to the amount of an active agent, for example, a therapeutically effective substance, such as a cyclooxygenase inhibitor, an NF-kB inhibitor, or an antiirritant, required to be administered in order to induce a desired result in the patient. That result may be alleviation or amelioration (complete or partial) of the symptoms or condition of irritation, pain, tingling, redness or other discoloration of a healed wound, an improvement in the size and appearance of the healed wound, or any other desired improvement in the affected area of skin.

As used herein, the term "therapeutically effective amount" may also refer to the quantity of active agent or therapeutically effective substance, the administration of which results in improvement in the size, appearance, or condition of a healed wound, where little or no improvement would occur in the absence of the active agent. Typically, the active agent is administered for a sufficient period of time to achieve the desired therapeutic effect.

Therapeutic efficacy may be determined as described herein and by using standard pharmacological procedures in experimental animals.

The active ingredient of an embodiment of the invention, together with a conventional adjuvant, carrier, or diluent, may thus be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use, or in the form of aerosol formulations for inhalation therapy. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed. Further details on techniques for formulation and administration may be found in the latest edition of *Remington's Pharmaceutical Sciences* (Maack Publishing Co., Easton, Pa.).

When desired, compositions adapted to give sustained release of the active ingredient may be employed.

The dose administered is adjusted to the size and severity of the healed wound or affected area of skin, the age, weight and condition of the individual being treated, as well as the route of administration, dosage form and regimen, and the result desired. The exact dosage should of course be determined by the practitioner.

The active ingredient can be administered in one or several doses per day. In one embodiment, it is presently contemplated that, for therapeutic treatments, at least one composition of the present invention, such as a cyclooxygenase inhibitor or an antiirritant, can be administered in an amount comprising from about 1 microgram to about 3000 micrograms, from about 10 micrograms to about 2000 micrograms, from about 20 micrograms to about 1000 micrograms, or from about 40 micrograms to about 400 micrograms per square centimeter of treated tissue.

In another embodiment, for therapeutic treatments, at least one NF-kB inhibitor is administered in an amount comprising from about 1 microgram to about 2000 micrograms, from about 10 micrograms to about 1000 micrograms, or from about 40 micrograms to about 400 micrograms per square centimeter of treated tissue. The amount of composition of the present invention can be administered by any suitable method of administration, including, but not limited to, topical application, subcutaneous or parenteral administration, oral administration, administration by inhalation, and by combinations of these methods.

In one embodiment, the amount of composition of the present invention can be included in an amount from about 1 percent by weight to about 75 percent by weight, from about 5 percent to about 50 percent by weight, or from about 10 percent to about 40 percent by weight, in a thermal insulating material. In a particular embodiment, the composition of the present invention is included in an amount of about 40 percent by weight in a thermal insulating material. The composition can be administered topically to deliver an amount comprising from about between about 1 microgram to about 3000 micrograms, from about 10 micrograms to about 2000 micrograms, from about 20 micrograms to about 1000 micrograms, or from about 40 micrograms to about 400 micrograms, for example, of NF-kB inhibitor or cyclooxygenase inhibitor, per square centimeter of treated tissue. For example, in one embodiment, a healed wound is treated by contacting the healed wound with a hydrogel, the hydrogel including acetylsalicylic acid present in an amount up to about 40 percent of the weight of the hydrogel; the amount of acetylsalicylic acid that is administered comprises from about 1 microgram to about 2000 micrograms, or from about 40 micrograms to about 400 micrograms of acetylsalicylic acid per square centimeter of treated tissue; and the surface temperature of the healed wound is elevated by the hydrogel from about 0.5°C to about 5°C.

In another embodiment, the composition of the present invention can be administered as a composition comprising from about 0.01 percent to about 80 percent, from about 0.05 percent to about 50 percent, or from about 0.1 percent to about 10 percent of said composition in admixture with a pharmaceutically acceptable carrier.

In another embodiment, a healed wound is treated by contacting the healed wound with a thermal insulating material including an effective amount of at least one antiirritant compound in a suitable pharmaceutical carrier. The thermal insulating material may include a hydrogel. Examples of an antiirritant compound or substance that relieves skin irritation that can be used according to this embodiment of the invention include: glyceryl monooleate, diphenhydramine, calamine, and a C₃-C₄ diol. The amount of antiirritant compound used in this embodiment comprises from about 1 microgram to about 2000 micrograms, or from about 40 micrograms to about 400 micrograms of antiirritant compound per square centimeter of treated tissue. The thermal insulating material elevates the surface temperature of the healed wound, and is allowed to remain in contact with the affected area of skin.

The amount of cyclooxygenase inhibitor, NF-kB inhibitor, antiirritant, or combination thereof that is administered, and the dosing regimen used, will, of course, be dependent on the particular drug selected, the route or routes of administration employed, the age and general condition of the subject being treated, the severity of the subject's condition, and the judgment of the prescribing physician. Generally, the daily dosage when administered topically or by injection will be determined by, among other factors, the dosage which may be given by some other mode of administration, such as oral. Alternatively, a large initial loading dose can be used to achieve effective levels of the agent, and can be followed by smaller doses to maintain those levels.

In another embodiment, the invention comprises a kit, or packaged assembly, in the form of a consumer package or prescription package which provides the necessary ingredients described herein together with directions on how to combine the ingredients to make a hydrogel for use in treatment of a healed wound (e.g., to treat a condition of scar irritation, or to reduce hypertrophic scarring). In one embodiment, a kit may include ingredients that can be co-administered with a hydrogel by mixing one or more ingredients with the hydrogel for topical application, or for another mode of administration such as oral.

In one embodiment, a kit for improving the size and appearance of a healed wound can include a composition of the present invention, such as a cyclooxygenase inhibitor, an NF-kB inhibitor, or an antiirritant and a hydrogel. Such a kit may further include a sterile solution (e.g., saline, water) for mixing with the composition of the present invention. The composition can be applied to the healed wound and covered with the hydrogel. In another embodiment, a kit may include a hydrogel that includes a composition of the present invention such as a cyclooxygenase inhibitor or an NF-kB inhibitor.

In another embodiment, a kit may include a hydrogel and a composition comprising up to about 35 percent of each of the following: salicylic acid or a derivative thereof; acetylsalicylic acid or a derivative thereof; a compound selected from aluminum hydroxide, aluminum zirconium trichlorohydrex, and other metallic anti-naicrobials; a compound selected from diphenhydramine and other anti-pruritic agents; a compound selected from ibuprofen and other non-steroidal agents specifically inhibiting prostaglandin E2; and a compound selected from non-steroidal agents specifically inhibiting cyclooxygenase 2.

In a particular embodiment, a kit can include a hydrogel and a composition comprising about 2 percent to about 5 percent of salicylic acid or a derivative thereof; about 2 percent to about 5 percent of acetylsalicylic acid or a derivative thereof; about 2 percent to about 5 percent of a compound such as aluminum hydroxide, aluminum zirconium trichlorohydrex, and other metallic anti-microbials; about 2 percent to about 5 percent of a compound such as diphenhydramine and other anti-pruritic agents; about 2 percent to about 5 percent of a compound such as ibuprofen and other non-steroidal agents specifically inhibiting prostaglandin E2; and about 2 percent to about 5 percent of a compound such as a non-steroidal agent specifically inhibiting cyclooxygenase 2, and mixtures thereof.

In another embodiment the kit may include, in addition to active ingredients and other materials for topical administration, a cyclooxygenase inhibitor and/or an antiirritant such as diphenhydramine for oral administration.

In one embodiment, a kit may include a hydrogel composition including up to 20% by weight polyethylene glycol (PEG) with up to 95% by weight sterile water. Because PEG is most efficiently sterilized when in powder form, the preparation of the hydrogel by the consumer or patient increases the quality of available gels and reduces the cost of the scar control therapy by leaving the addition of water to the PEG powder until immediately prior to use.

In addition, a kit-based preparation of hydrogel which includes, in one embodiment, an anti-pruritic ingredient and/or a cyclooxygenase inhibitor and/or an NF-kB inhibitor, and/or a topical antimicrobial, permits a consumer or patient access to a therapy individually tailored or designed to the size and severity of the irritated scar condition. The components can be prescribed by a treating clinician or can be self-selected based on the patient's current assessment of the scar condition.

A healed wound control kit according to an embodiment can include devices such as "control top" panties or other garments for use with scars of the lower abdominal skin, stretch bandages (e.g. elastic-type sports wraps or ace wraps), and gloves to keep the hydrogel affixed to the affected area of skin.

Materials in a kit may also include: protective gloves for user to wear during hydrogel preparation; miniature timer to allow user to time the hydrogel preparation; miniature spatula to smooth or stir hydrogel ingredients; hydrogel "tray" or "mold" of depth not to exceed 0.5 cm, and length and width varying depending on the amount of hydrogel to be prepared by user; a ruler either in paper, plastic, or tape form, to allow user to measure affected skin to be treated and to choose correctly the size and amount of hydrogel to be prepared.

An example of a method according to one embodiment of using a kit may include reading directions prior to kit use; using included ruler to measure affected area of skin; comparing scar size to hydrogel preparation table for ingredients and recipe or to prescription or clinician recommendation; using spatula to mix recommended amount of water with PEG ingredient in amount specific to scar size; placing PEG and water mixture in mold or tray and timing; adding active ingredients according to prescription or as recommended by clinician or by personal preference; applying resulting hydrogel to affected area of skin and securing the hydrogel to the skin. A method according to an embodiment may also include coadministering other compositions depending on prescription, as recommended by clinician, or according to personal preference.

A kit according to an embodiment may include ingredients or components for one treatment or may include ingredients or components for multiple treatments. A kit according to an embodiment may include a combination of medications and devices tailored to a patient's irritated scar condition.

### Exemplification

### Example 1: Patient SS

The patient's chief complaint was local irritation with clothing use and itching from scars located on the anterior upper chest. The patient underwent a medical history and physical examination. Examination was significant for hypertrophic acne scar of approximately 1 cm by 0.5 cm (long axis by short axis) on the anterior chest, the scar notable for redness, and tenderness to palpation. Materials and Method: After review of clinical options, the patient was prescribed the combination of nabumetone (oral dose ranging from 250-500 mg at least 30 minutes before meals one time each day) and diphenhydramine (oral dose ranging from 25 to 50 mg) before bedtime. These drugs were initiated in the lowest dose noted and increased 6 weeks later. Hydrogel (AVOGEL®) or a gel (Elastogel®) was co-administered with the drugs and topically applied as gel sheeting ranging from 0.15 to 0.25 cm in thickness extending approximately 2 cm beyond the contours of the treated scar. To avoid the use of adhesives, the hydrogel was held in place with a spandex upper chest garment. Treatment continued each day for a period of 15-18 hours for a duration of 13 months. Clinical progress was followed using photographic documentation, patient self-report, and physical examination. The patient self-report instrument incorporating an itching scale was completed to assess treatment progress using a 10 point scale. RESULTS: The itching scale showed a decrease in the experience of scar irritation leading to itching across the therapeutic period from an initial self-report of a rating of 8 in November 1998 to a rating of 0 in December 1999. Follow-up photographs showed that the decrease in irritation was accompanied by a reduction of approximately 50% in the degree of redness. On serial photographs, the contours of the treated scar changed from that of rough, raised, irregular texture, to a smooth, flatter and more regular texture. Physical examination revealed a flattening scar with less redness and less tenderness.

### Example 2: Patient MB

The patient's chief complaint was irritation with daily grooming and itching from scars located on the face. The patient underwent a medical history and physical examination. Examination revealed a 1.5 cm length oval shaped tender hypertrophic tender scar located at the left mid-face lateral to the nasal-labial fold, the scar raised from the planar surface of the surrounding skin by approximately 2 mm.
Materials and Methods: After review of clinical options, the patient was prescribed the combination of nabumetone and hydrogel co-administered for a minimum of 15-18 hours each day for a period of 7 weeks duration. Nabumetone (oral dose ranging from 250 to 500 mg at least 30 minutes before meals one time each day) was initiated in the lowest dose and increased at 6 weeks. Hydrogel sheeting (AVOGEL®) ranging from 0.15 to 0.25 cm in thickness was topically applied, extending approximately 2 cm beyond the contours of the scar and held in place with facial chin strap. Clinical progress was followed using photographic and ultrasound imaging as well as a patient self-report. RESULTS: On examination, the treated scar was observed to reduce in size 50% and to revert to a color tone more closely matching adjacent skin, with less redness and tenderness on exam. Color changes were documented by photographs. Ultrasound images documented the scar size change. Patient self-report indicated improved comfort.

### Example 3: Patient 25

The patient's chief complaint was an irritated scar of the forearm for more than two years. The patient underwent a medical history and physical examination. On examination, a raised irregularly contoured errythematous hypertrophic scar was observed.
Materials and Methods: After review of clinical options, the patient was prescribed nabumetone for 4 weeks. Oral nabumetone was started at a dose 250 mg at least 30 minutes before meals one time each day. After 4 weeks, while oral nabumetone was continued, hydrogel sheeting (AVOGEL®) ranging from 0.15 to 0.25 cm in thickness was topicallyco-administered, extending approximately 2 cm beyond the contours of the scar and held in place with a simple gauze wrap. The combination of nabumetone and hydrogel was used for 15-18 hours each day for a period of 8 weeks. Clinical progress was followed using photographic imaging as well as patient self-report. RESULTS: On examination, the treated scar became flatter and reverted in color to skin tones closely matching adjacent skin. Irregular contours faded. On photographic documentation, the scar was not observable at treatment day 60. Patient self-report indicated improved comfort.

### Example 4: Patient JV

The patient's chief complaint was difficulty sleeping or resting because of a post-operative orthopoedic right leg scar that was irritated and raised. The patient underwent a medical history and physical examination. Notable medication use was a 325 mg tablet of acetylsalicylic acid orally administered to prevent thromboembolic post-operative complications. On examination, a 15 cm raised erythematous post-operative scar of 15 cm length and approximately 3 mm width was noted, with exquisite sensitivity to examination and evidence of excoriation. At the time of exam, the patient denied itching.
Materials and Methods: After review of clinical options, the patient was prescribed the combination of 2% salicylic acid applied topically using a roll-on applicator under hydrogel (AVOGEL®) 15-18 hours each day for 12 weeks duration. To avoid the use of adhesives, an ace wrap bandage was used to hold the gel - salicylic acid combination in place. Gel plus salicylic acid co-administration was monitored by a daily patient diary. Photographs and patient self report were used to assess treatment results. Itching complaints were followed using a 10 point itching scale. Pre-treatment itching scale was entered as 0, reflecting onset of aspirin use prior therapy. RESULTS: Compliance with treatment regimen was documented daily. Photographs revealed shortened measure of the scar consistent with scar contracture by 25%. On examination, the scar contours appeared to have flattened and the scar color appeared lighter than in pre-treatment. No evidence of itching or irritation was noted. Itching symptoms were reported at 0. Patient reported improved sleep with reduction in scar irritation.

### Example 5: Patient JC

The patient's chief complaint was a burning itchy sensation in a closed post-operative caesarean section scar. The patient underwent a medical history and physical examination. On examination, an 18 cm irritated lower abdominal vertical healed incision was noted, with evidence of excoriation.
Methods and Materials: After review of clinical options, the patient was prescribed the combination of 2% salicylic acid applied topically using a roll-on applicator under hydrogel (AVOGEL®), 15-18 hours each day for 12 weeks duration. To avoid the use of adhesives, a control top stretch panty was used to hold the gel - salicylic acid combination in place. Gel plus salicylic acid co-administration was monitored by a daily patient diary. Photographs and patient self report were followed to assess treatment results. Itching complaints were followed using a 10 point itching scale. Pre-treatment itching scale was entered as 6, with the comment that itching bothered the patient "severely, badly." RESULTS: Compliance with treatment regimen was documented daily. Photographs revealed reduced redness and local irritation at scar site. On examination, the scar contours appeared to have become more regular and scar color faded. No evidence of itching or irritation was noted. Itching symptoms were reported at 3 at the 4^{th} week of treatment when the patient noted she was "not breaking open her skin itching now." Scar irritation and pruritis was reduced to infrequent at the end of treatment after 20 weeks.

## Claims

1. Use of at least one cyclooxygenase inhibitor for the manufacture of a medicament for reducing the size and improving the appearance of a closed wound, wherein the closed wound is caused by appearance of a hypertrophic or keloid scar on a wound that is closed after an open wound has been re-epithelialized, and wherein the wound is selected from the group consisting of: a wound caused by laceration: a wound caused by avulsion; a wound caused by burn; a wound caused by surgery, a wound caused by chemical facial peel; and a wound caused by accident.

2. The use of Claim 1 wherein the at least one cyclooxygenase inhibitor is selected from salicylic acid and salts thereof, acetylsalicylic acid and salts thereof, substituted or unsubstituted aralkyl, allyl, and substituted or unsubstituted, linear, branched, or cyclic alkyl esters of acetylsalicylic acid; ibuprofen; celecoxib; rofecoxib; flufenamic acid; indomethacin; nabumetone; naproxen; pharmaceutically acceptable salts thereof; and blends thereof.

3. The use of Claim 1. wherein the ester of acetylsalicylic acid is selected from methyl acetylsalicylate, ethyl acetylsalicylate, and benzyl acetylsalicylate.

4. Use of a cyclooxygenase inhibitor for the manufacture of a medicament for reducing the size and improving the appearance of a closed wound, wherein the cyclooxygenase inhibitor is present in a hydrogel.

5. Use of at least one NF-kB inhibitor for the manufacture of a medicament, for reducing the size and improving the appearance of a closed wound.

6. The use of any one of claims 1, 2, and 5 wherein the cyclooxygenase inhibitor or NF-kB inhibitor is present in a thermal insulating material.

7. The use of Claim 5 or Claim 6 wherein the NF-kB inhibitor is selected from salicylic acid; salts of salicylic acid; aryl, substituted or unsubstituted aralkyl, allyl, and substituted or unsubstituted, linear, branched, or cyclic alkyl esters of salicylic acid; sulindac sulfide; sulindac sulfone; sulfasalazine; pharmaceutically acceptable salts thereof; and blends thereof.

8. The use of any one of claims 1, 2, 3, 5, 6, and 7 wherein the cyclooxygenase inhibitor or NF-kB inhibitor is administered using a route of administration selected from topical application to the closed wound, oral, injection, and combinations thereof.

9. The use of claim 1 or claim 5 wherein the medicament comprises a pharmaceutical carrier which includes one or more substances that relieve skin irritation when the cyclooxygenase or NF-kB inhibitor is topically administered to the closed wound.

10. The use of claim 6 wherein (a) the cyclooxygenase inhibitor or NF-kB inhibitor is present in an amount up to 40 percent of the weight of the thermal insulating material; or (b) the thermal insulating material comprises a sponge; or (c) the cyclooxygenase inhibitor or NF-kB inhibitor is administered as a composition comprising from 0.1 to 10 percent by weight of said inhibitor in admixture with a pharmaceutically acceptable carrier.

11. Use of an NF-kB inhibitor for the manufacture of a medicament for reducing the size and improving the appearance of a closed wound, wherein the NF-kB inhibitor is present in a hydrogel.

12. Use of a thermal insulating material including at least one antiirritant compound and at least one cyclooxygenase or NF-kB inhibitor for the manufacture of a medicament for reducing the size and improving the appearance of a closed wound.

13. The use of Claim 9 or Claim 12 wherein the antiirritant material includes at least one substance selected from diphenhydramine, calamine, and a C₃-C₄ diol.

14. Use of a hydrogel including acetylsalicylic acid for the manufacture of a medicament for reducing the size and improving the appearance of a closed wound, the hydrogel being effective to elevate the surface temperature of the closed wound.

15. The use of any one of the preceding claims wherein the cyclooxygenase inhibitor, NF-kB inhibitor, or antiirritant material or acetylsalicylic acid is administered with a suitable pharmaceutical carrier.

16. The use of any one of the preceding claims wherein the amount of cyclooxygenase inhibitor, NF-kB inhibitor, antiirritant material or acetylsalicylic acid that is administered comprises from 40 micrograms to 400 micrograms of inhibitor material or acetylsalicylic acid per square centimetre of treated tissue.

17. The use of any one of the preceding claims wherein the surface temperature of the closed wound is elevated from 0.5°C to 5°C.

18. The use of Claim 16 wherein the acetylsalicylic acid is present in an amount up to 40 percent of the weight of the hydrogel.

19. The use of any one of the preceding claims wherein the closed wound is a hypertrophic scar, a keloid scar, a Dupuytren's contracture, a reactive scar, an excessive post-operative scar, or a fibrotic scar.

20. The use of any one of the preceding claims wherein a deodorant agent is included in the medicament.

21. The use of claim 20, wherein the deodorant agent is selected from aluminum zirconium trichlorohydrex and zinc acetate.

22. Use of a cyclooxygenase inhibitor or an NF-kB inhibitor for the manufacture of a medicament for preventing or treating a condition caused by the appearance of a hypertrophic or a keloid scar on a closed wound, in combination with a substance that relieves skin irritations, an antimicrobial agent, and thermal insulating material.

23. A kit for reducing the size and improving the appearance of a closed wound comprising (a) a cyclooxygenase inhibitor or (b) an NF-kB inhibitor and a hydrogel.

24. A kit for reducing the size and improving the appearance of a closed wound comprising a hydrogel that includes (a) a cyclooxygenase inhibitor, or (b) an NF-kB inhibitor.

25. A kit according to Claim 23 further comprising a sterile solution for mixing with the cyclooxygenase inhibitor or NF-kB inhibitor.

26. A kit for reducing the size and improving the appearance of a closed wound including a hydrogel and a composition comprising:
i) 2 percent to 5 percent of salicylic acid or a derivative thereof;
ii) 2 percent to 5 percent of acetylsalicylic acid or a derivative thereof;
iii) 2 percent to 5 percent of a compound selected from ibuprofen and other non-steroidal agents specifically inhibiting prostaglandin E2; and
iv) 2 percent to 5 percent of a compound selected from non-steroidal agents specifically inhibiting cyclooxygenase 2;
v) 2 percent to 5 percent of a compound selected from aluminum hydroxide, aluminum zirconium trichlorohydrex, and other metallic anti-microbials;
vi) 2 percent to 5 percent of a compound selected from diphenhydramine and other anti-pruritic agents; and
vii) mixtures thereof.

27. A kit according to Claim 23, further including (a) an anti-pruritic compound and an anti-microbial agent; or (b) at least one device for affixing the hydrogel to an affected area of skin.

28. A kit according to Claim 24, further including (a) a cyclooxygenase inhibitor for oral administration, or (b) diphenhydramine for oral administration.

## Patentansprüche

1. Verwendung mindestens eines Cyclooxygenase-Inhibitors zur Herstellung eines Medikaments zur Reduktion der Größe und zur Verbesserung des Erscheinungsbildes einer geschlossenen Wunde, wobei die geschlossene Wunde durch das Auftreten einer hypertrophen Narbe oder eines Narbenkeloids auf einer Wunde, die nach der Reepitelisierung einer offenen Wunde geschlossen wird, bewirkt wird und wobei die Wunde aus der Gruppe ausgewählt wird, bestehend aus: einer durch Riß verursachten Wunde; einer durch Ausreißen verursachten Wunde; einer durch Verbrennung verursachten Wunde; einer durch Operation verursachten Wunde; einer durch chemisches Gesichtspeeling verursachten Wunde; und einer durch Unfall verursachten Wunde.

2. Verwendung nach Anspruch 1, wobei der mindestens eine Cyclooxygenase-Inhibitor aus Salicylsäure und Salzen davon, Acetylsalicylsäure und Salzen davon, substituierten oder unsubstituierten Aralkyl-, Allyl-, und substituierten oder unsubstituierten linearen, verzweigten oder cyclischen Alkylestern von Acetylsalicylsäure; Ibuprofen; Celecoxib; Rofecoxib; Flufenaminsäure; Indomethacin; Nabumeton; Naproxen; pharmazeutisch verträglichen Salzen davon; und Mischungen davon ausgewählt ist.

3. Verwendung nach Anspruch 1, wobei der Ester von Acetylsalicylsäure aus Methylacetylsalicylat, Ethylacetylsalicylat und Benzylacetylsalicylat ausgewählt ist.

4. Verwendung eines Cyclooxygenase-Inhibitors zur Herstellung eines Medikaments zur Reduktion der Größe und zur Verbesserung des Erscheinungsbildes einer geschlossenen Wunde, wobei der Cyclooxygenase-Inhibitor in einem Hydrogel vorhanden ist.

5. Verwendung von mindestens einem NF-kβ-Inhibitor zur Herstellung eines Medikaments zur Reduktion der Größe und zur Verbesserung des Erscheinungsbildes einer geschlossenen Wunde.

6. Verwendung nach einem der Ansprüche 1, 2 und 5, wobei der Cyclooxygenase-Inhibitor oder NF-kβ-Inhibitor in einem wärmeisolierenden Material vorhanden ist.

7. Verwendung nach Anspruch 5 oder Anspruch 6, wobei der NF-kβ-Inhibitor aus Salicylsäure; Salzen von Salicylsäure; Aryl-, substituierten oder unsubstituierten Aralkyl-, Allyl- und substituierten oder unsubstituierten linearen, verzweigten oder cyclischen Alkylestern von Salicylsäure; Sulindacsulfid; Sulindacsulfon; Sulfasalazin; pharmazeutisch verträglichen Salzen davon; und Mischungen davon ausgewählt ist.

8. Verwendung nach einem der Ansprüche 1, 2, 3, 4, 5, 6, und 7, wobei der Cyclooxygenase-Inhibitor oder NF-kβ-Inhibitor unter Verwendung eines Verabreichungsweges verabreicht wird, der, aus topischer Verabreichung auf die geschlossene Wunde, oral, Injektion und Kombinationen davon ausgewählt wird.

9. Verwendung nach Anspruch 1 oder Anspruch 5, wobei das Medikament einen pharmazeutischen Träger umfasst, der eine oder mehrere Substanzen einschließt, die Hautreizung lindern, wenn der Cyclooxygenase- oder NF-kβ-Inhibitor topisch auf die geschlossene Wunde verabreicht wird.

10. Verwendung nach Anspruch 6, wobei (a) der Cyclooxygenase-Inhibitor oder NF-kβ-Inhibitor in einer Menge bis zu 40 % des Gewichts des wärmeisolierenden Materials vorhanden ist; oder (b) das wärmeisolierende Material einen Schwamm einschließt; oder (c) der Cyclooxygenase-Inhibitor oder NF-kβ-Inhibitor als Zusammensetzung verabreicht wird, die 0;1 bis 10 Gew.-% des Inhibitors im Gemisch mit einem pharmazeutisch verträglichen Träger umfasst.

11. Verwendung eines NF-kβ-Inhibitors zur Herstellung eines Medikaments zur Reduktion der Größe und zur Verbesserung des Erscheinungsbildes einer geschlossenen Wunde, wobei der NF-kβ-Inhibitor in einem Hydrogel vorhanden ist.

12. Verwendung eines wärmeisolierenden Materials, das mindestens einen Reizhemmer und mindestens einen Cyclooxygenase- oder einen NF-kβ-Inhibitor einschließt, zur Herstellung eines Medikaments zur Reduktion der Größe und zur Verbesserung des Erscheinungsbildes einer geschlossenen Wunde.

13. Verwendung nach Anspruch 9 oder Anspruch 12, wobei das reizhemmende Material mindestens eine Substanz einschließt, die aus Diphenhydramin, Calamin und einem C₃-C₄-Diol ausgewählt ist.

14. Verwendung eines Hydrogels, das Acetylsalicylsäure einschließt, zur Herstellung eines Medikaments zur Reduktion der Größe und zur Verbesserung des Erscheinungsbildes einer geschlossenen Wunde, wobei das Hydrogel zur Erhöhung der Oberflächentemperatur der geschlossenen Wunde wirksam ist.

15. Verwendung nach einem der vorhergehenden Ansprüche, wobei der Cyclooxygenase-Inhibitor, der NF-kβ-Inhibitor oder das reizhemmende Material oder die Acetylsalicylsäure mit einem geeigneten pharmazeutischen Träger verabreicht wird.

16. Verwendung nach einem der vorhergehenden Ansprüche, wobei die Menge an Cyclooxygenase-Inhibitor, NF-kβ-Inhibitor, reizhemmendem Material oder Acetylsalicylsäure, die verabreicht wird, 40 µg bis 400 µg Inhibitormaterial oder Acetylsalicylsäure pro cm² von behandeltem Gewebe umfasst.

17. Verwendung nach einem der vorhergehenden Ansprüche, wobei die Oberflächentemperatur der geschlossenen Wunde um 0,5 °C bis 5 °C erhöht wird.

18. Verwendung nach Anspruch 16, wobei die Acetylsalicylsäure in einer Menge bis zu 40 Gew.-% des Hydrogels vorhanden ist.

19. Verwendung nach einem der vorhergehenden Ansprüche, wobei die geschlossene Wunde eine hypertrophe Narbe oder ein Narbenkeloid, eine Dupuytren-Kontraktur, eine reaktive Narbe, eine überschießende postoperative Narbe oder eine fibrotische Narbe ist.

20. Verwendung nach einem der vorhergehenden Ansprüche, wobei ein desodorierendes Mittel in das Medikament eingeschlossen wird.

21. Verwendung nach Anspruch 20, wobei das desodorierende Mittel aus Aluminiumzirkoniumtrichlorohydrex und Zinkacetat ausgewählt ist.

22. Verwendung eines Cyclooxygenase-Inhibitors oder eines NF-kβ-Inhibitors zur Herstellung eines Medikaments zur Verhinderung oder Behandlung eines durch das Aussehen einer hypertrophen Narbe oder eines Narbenkeloids auf einer geschlossenen Wunde verursachten Zustands in Kombination mit einer Substanz, die Hautreizungen lindert, einem antimikrobiellen Mittel und einem wärmeisolierenden Mittel.

23. Kit zur Reduktion der Größe und zur Verbesserung des Erscheinungsbildes einer geschlossenen Wunde, das (a) einen Cyclooxygenase-Inhibitor oder (b) einen NF-kβ-Inhibitor und ein Hydrogel umfasst.

24. Kit zur Reduktion der Größe und zur Verbesserung des Erscheinungsbildes einer geschlossenen Wunde, das ein Hydrogel einschließt, das (a) einen Cyclooxygenase-Inhibitor oder (b) einen NF-kβ-Inhibitor umfasst.

25. Kit nach Anspruch 23, das weiterhin eine sterile Lösung zum Mischen mit dem Cyclooxygenase-Inhibitor oder dem NF-kβ-Inhibitor umfasst.

26. Kit zur Reduktion der Größe und zur Verbesserung des Erscheinungsbildes einer geschlossenen Wunde, das ein Hydrogel und eine Zusammensetzung einschließt, umfassend:
i) 2 bis 5 % Salicylsäure oder ein Derivat davon;
ii) 2 bis 5 %. Acetylsalicylsäure oder ein Derivat davon;
iii) 2 bis 5 % einer Verbindung, ausgewählt aus Ibuprofen und anderen nicht steroiden Mitteln, die spezifisch Prostaglandin E2 hemmen; und
iv) 2 bis 5 % einer Verbindung, ausgewählt aus nicht steroiden Mitteln, die spezifisch Cyclooxygenase 2 hemmen;
v) 2 bis 5 % einer Verbindung, ausgewählt aus Aluminiumhydroxid, Aluminiumzirkoniumtrichlorohydrex, und anderen metallischen Mikrobiziden;
vi) 2 bis 5 % einer Verbindung, ausgewählt aus Diphenhydramin und anderen antipruriginösen Mitteln; und
vii) Gemische davon.

27. Kit nach Anspruch 23, der weiterhin (a) eine antipruriginöse Verbindung und ein antimikrobielles Mittel; oder (b) mindestens eine Vorrichtung zum Anbringen des Hydrogels auf einem betroffenen Hautbereich umfasst.

28. Kit nach Anspruch 24, der weiterhin (a) einen Cyclooxygenase-Inhibitor zur oralen Verabreichung oder (b) Diphenhydramin zur oralen Verabreichung einschließt.

## Revendications

1. Utilisation d'au moins un inhibiteur de la cyclooxygénase pour la fabrication d'un médicament destiné à réduire la taille et à améliorer l'aspect d'une blessure fermée, dans laquelle la blessure fermée est provoquée par l'apparition d'une cicatrice hypertrophique ou chéloïde sur une blessure qui est fermée après la réépithélialisation d'une blessure ouverte, et dans laquelle la blessure est choisie dans le groupe constitué par : une blessure provoquée par une lacération ; une blessure provoquée par une avulsion ; une blessure provoquée par une brûlure ; une blessure provoquée par une opération chirurgicale ; une blessure provoquée par un peeling facial chimique ; et une blessure provoquée par un accident.

2. Utilisation selon la revendication 1, dans laquelle l'au moins un inhibiteur de la cyclooxygénase est choisi parmi l'acide salicylique et les sels de celui-ci, l'acide acétylsalicylique et les sels de celui-ci, les esters d'aralkyle substitué ou non substitué, d'allyle et les esters d'alkyle linéaires, ramifiés ou cycliques substitué ou non substitué d'acide acétylsalicylique ; l'ibuprofene; le célécoxib ; le rofécoxib ; l'acide flufénamique; l'indométhacine ; la nabumétone ; la naproxène ; les sels pharmaceutiquement acceptables de ceux-ci ; et les mélanges de ceux-ci.

3. Utilisation selon la revendication 1, dans laquelle l'ester d'acide acétylsalicylique est choisi parmi l'acétylsalicylate de méthyle, l'acétylsalicylate d'éthyle et l'acétylsalicylate de benzyle.

4. Utilisation d'un inhibiteur de la cyclooxygénase pour la fabrication d'un médicament destiné à réduire la taille et à améliorer l'aspect d'une blessure fermée, dans laquelle l'inhibiteur de la cyclooxygénase est présent dans un hydrogel.

5. Utilisation d'au moins un inhibiteur NF-kB pour la fabrication d'un médicament destiné à réduire la taille et à améliorer l'aspect d'une blessure fermée.

6. Utilisation selon l'une quelconque des revendications 1, 2 et 5, dans laquelle l'inhibiteur de la cyclooxygénase ou l'inhibiteur NF-kB est présent dans un isolant thermique.

7. Utilisation selon la revendication 5 ou la revendication 6, dans laquelle l'inhibiteur NF-kB est choisi parmi l'acide salicylique ; les sels d'acide salicylique ; les esters d'aryle, d'aralkyle substitué ou non substitué, d'allyle et les esters d'alkyle linéaires, ramifiés ou cycliques substitué ou non substitué d'acide salicylique ; le sulfure de sulindac ; la sulfone de sulindac ; le sulfasalazine ; les sels pharmaceutiquement acceptables de ceux-ci ; et les mélanges de ceux-ci

8. Utilisation selon l'une quelconque des revendications 1, 2, 3, 5, 6 et 7, dans laquelle l'inhibiteur de la cyclooxygénase ou l'inhibiteur NF-kB est administré en utilisant une voie d'administration choisie parmi l'application topique sur la blessure fermée, l'administration orale, l'injection et les combinaisons de celles-ci.

9. Utilisation selon la revendication 1 ou la revendication 5, dans laquelle le médicament comprend un excipient pharmaceutique qui comprend une ou plusieurs substances qui apaisent l'irritation cutanée lorsque l'inhibiteur de la cyclooxygénase ou l'inhibiteur NF-kB est administré par voie topique sur la blessure fermée.

10. Utilisation selon la revendication 6, dans laquelle (a) l'inhibiteur de la cyclooxygénase ou l'inhibiteur NF-kB est présent en une quantité pouvant atteindre 40 pour cent du poids de l'isolant thermique ; ou (b) l'isolant thermique comprend une éponge ; ou (c) l'inhibiteur de la cyclooxygénase ou l'inhibiteur NF-kB est administré sous la forme d'une composition comprenant de 0,1 à 10 pour cent en poids dudit inhibiteur en mélange avec un excipient pharmaceutiquement acceptable.

11. Utilisation d'un inhibiteur NF-kB pour la fabrication d'un médicament destiné à réduire la taille et à améliorer l'aspect d'une blessure fermée, dans laquelle l'inhibiteur NF-kB est présent dans un hydrogel.

12. Utilisation d'un isolant thermique comprenant au moins un composé anti-irritant et au moins un inhibiteur de la cyclooxygénase ou un inhibiteur NF-kB pour la fabrication d'un médicament destiné à réduire la taille et à améliorer l'aspect d'une blessure fermée.

13. Utilisation selon la revendication 9 ou la revendication 12, dans laquelle la matière anti-irritante comprend au moins une substance choisie parmi la diphénhydramine, la calamine et un diol en C₃ à C₄.

14. Utilisation d'un hydrogel comprenant de l'acide acétylsalicylique pour la fabrication d'un médicament destiné à réduire la taille et à améliorer l'aspect d'une blessure fermée, l'hydrogel étant efficace pour augmenter la température de surface de la blessure fermée.

15. Utilisation selon l'une quelconque des revendications précédentes, dans laquelle l'inhibiteur de la cyclooxygénase, l'inhibiteur NF-kB, la matière anti-irritante ou l'acide acétylsalicylique est administré avec un excipient pharmaceutique approprié.

16. Utilisation selon l'une quelconque des revendications précédentes, dans laquelle la quantité d'inhibiteur de la cyclooxygénase, d'inhibiteur NF-kB, de matière anti-irritante ou d'acide acétylsalicylique qui est administrée comprend de 40 microgrammes à 400 microgrammes de matière inhibitrice ou d'acide acétylsalicylique par centimètre carré de tissu traité.

17. Utilisation selon l'une quelconque des revendications précédentes, dans laquelle la température de surface de la blessure fermée est augmentée de 0,5 °C à 5 °C.

18. Utilisation selon la revendication 16, dans laquelle l'acide acétylsalicylique est présent en une quantité pouvant atteindre 40 pour cent du poids de l'hydrogel.

19. Utilisation selon l'une quelconque des revendications précédentes, dans laquelle la blessure fermée est une cicatrice hypertrophique, une cicatrice chéloïde, la maladie de Dupuytren, une cicatrice réactive, une cicatrice postopératoire excessive ou une cicatrice fibrotique.

20. Utilisation selon l'une quelconque des revendications précédentes, dans laquelle un agent déodorant est inclus dans le médicament.

21. Utilisation selon la revendication 20, dans laquelle l'agent déodorant est choisi parmi le trichlorohydrex d'aluminium et de zirconium et l'acétate de zinc.

22. Utilisation d'un inhibiteur de la cyclooxygénase ou d'un inhibiteur NF-kB pour la fabrication d'un médicament destiné à prévenir ou traiter une condition provoquée par l'apparition d'une cicatrice hypertrophique ou d'une cicatrice chéloïde sur une blessure fermée, en combinaison avec une substance qui apaise les irritations cutanées, un agent antimicrobien et un isolant thermique.

23. Kit de réduction de la taille et d'amélioration de l'aspect d'une blessure fermée comprenant (a) un inhibiteur de la cyclooxygénase ou (b) un inhibiteur NF-kB et un hydrogel.

24. Kit de réduction de la taille et d'amélioration de l'aspect d'une blessure fermée comprenant un hydrogel qui comprend (a) un inhibiteur de la cyclooxygénase ou (b) un inhibiteur NF-kB.

25. Kit selon la revendication 23, comprenant en outre une solution stérile pour un mélange avec l'inhibiteur de la cyclooxygénase ou l'inhibiteur NF-kB

26. Kit de réduction de la taille et d'amélioration de l'aspect d'une blessure fermée comprenant un hydrogel et une composition comprenant :
i) 2 pour cent à 5 pour cent d'acide salicylique ou d'un dérivé de celui-ci;
ii) 2 pour cent à 5 pour cent d'acide acétylsalicylique ou d'un dérivé de celui-ci ;
iii) 2 pour cent à 5 pour cent d'un composé choisi parmi l'ibuprofène et d'autres agents non stéroïdiens inhibant spécifiquement la prostaglandine E2 ; et
iv) 2 pour cent à 5 pour cent d'un composé choisi parmi des agents non stéroïdiens inhibant spécifiquement la cyclooxygénase 2,
v) 2 pour cent à 5 pour cent d'un composé choisi parmi l'hydroxyde d'aluminium, le trichlorohydrex d'aluminium et de zirconium et d'autres antimicrobiens métalliques,
(vi) 2 pour cent à 5 pour cent d'un composé choisi parmi la diphénhydramine et d'autres agents antipruritiques, et
(vii) des mélanges de ceux-ci.

27. Kit selon la revendication 23, comprenant en outre (a) un composé antipruritique et un agent antimicrobien ; ou (b) au moins un dispositif de fixation de l'hydrogel à une zone de peau affectée.

28. Kit selon la revendication 24, comprenant en outre (a) un inhibiteur de la cyclooxygénase destiné à une administration orale ou (b) de la diphénhydramine destinée à une administration orale.
